# EUROPÄISCHE PATENTANMELDUNG

(11) **EP 0 678 278 A1**
(43) Veröffentlichungstag der Anmeldung: **25.10.1995**
(21) Anmeldenummer: 95107883.1
(22) Anmeldetag: 03.02.1993
(51) Int. Cl.: A61B 5/14, A61B 17/32

(54) **Lanzettenvorrichtung zum Punktieren der Haut**

(30) Priorität: 16.04.1992 DE 4212723
(62) Teilanmeldung aus: 93101620.8
(71) Anmelder: ARTA PLAST AB, S-135 48 Tyresö (SE)
(72) Erfinder: Steg, Hans-Henning, S-136 68 Haninge (SE); Seidl, Helmut, S-125 51 Alvsjö (SE)
(74) Vertreter: Niedmers, Ole, Dipl.-Phys.

(57) **Zusammenfassung**

Es wird eine Lanzettenvorrichtung (10) zum Punktieren der Haut (11) von Säugetieren, insbesondere des Menschen, vorgeschlagen, umfassend ein mit einer Handhabe (13) versehenes Steckelement (12) sowie ein mit einer Handhabe (15) versehenes Buchsenelement (14) zur inneren axial (16) verschiebbaren Aufnahme des Steckelements (12), wobei das Steckelement (12) an seinem von der Handhabe (13) weggerichteten freien Ende eine mit einem zugespitzten Ende versehene Lanzette (18) zum gezielten Eintritt in die Haut (11) aufweist. Die Handhabe (13) des Steckelements (12) ist wenigstens teilweise als federelastischer Körper nach Art einer Membran (20) ausgebildet, die die Lanzette (18) über das Steckelement (12) nach Überwindung eines membranseitig eingestellten Druckpunktes durch eine in axialer Richtung (34) auf das freie Buchsenende (21) hin einwirkende Kraft mit einem vorbestimmten Impuls aus der durch das Buchsenende (21) gebildeten Ebene (22) in die Haut (11) eintreten läßt.

## Beschreibung

Die Erfindung betrifft eine Lanzettenvorrichtung zum Punktieren der Haut von Säugetieren, insbesondere des Menschen, umfassend ein mit einer Handhabe versehene Steckelement sowie ein mit einer Handhabe versehenes Buchsenelement zur inneren axial verschiebbaren Aufnahme des Steckelementes, wobei das Steckelement an seinem von der Handhabe wegweisenden freien Ende eine mit einem zugespitzten Ende versehene Lanzette zum gezielten Eintritt in die Haut aufweist.

Lanzettenvorrichtungen zum gezielten Punktieren der Haut, insbesondere des Menschen, sind seit langem bekannt und werden in den unterschiedlichsten Ausführungsformen mit mehr oder weniger gutem Erfolg seit langem im ambulanten Bereich medizinischer Einrichtungen, in Krankenhäusern, bei Ärzten, bei Rote-Kreuz-Einrichtungen und auch bei Katastrophenschutzeinrichtungen und dergleichen angewendet, um beispielsweise geringere Mengen Blutes für Blutuntersuchungen zur Verfügung zu haben. Wesentliche Voraussetzung dieser Lanzettenvorrichtungen ist es, daß diese extrem kostengünstig bereitstellbar sein müssen, da sie in großen Mengen von den voraufgeführten Institutionen verwendet werden und dort auch in großen Mengen bereitgestellt werden müssen. Eine weitere wichtige Forderung an diese Lanzettenvorrichtungen ist die, daß zumindest die Teile der Lanzettenvorrichtung, die unmittelbar die Haut des Menschen durchstoßen, also unmittelbar Kontakt mit dem unter der Außenhaut liegenden Gewebe und dem Blut des Menschen haben, fortwährend bis zum Einsatz absolut steril gehalten werden müssen. In der Regel handelt es sich bei den Teilen der Lanzettenvorrichtung, die in das Gewebe in einer vorbestimmbaren Tiefe eindringen sollen, um Blutgefäße zu treffen und das Blut aus der Eintrittsöffnung der Wunde heraustreten zu lassen, um sogenannte Lanzetten, die aus einem im wesentlichen einen kreisförmigen Querschnitt aufweisenden Stahlkörper bestehen, der an seinem der zur punktierenden Haut weisenden Ende angespitzt ist.

In der Regel werden derartige Lanzettenvorrichtungen, nachdem mit ihnen einmal eine vorbestimmte Punktierfunktion ausgeübt worden ist, nicht wieder verwendet, da eine erneute Sterilisation der Lanzettenvorrichtung unvergleichlich viel kostspieliger wäre, als die Herstellung einer Lanzettenvorrichtung insgesamt. In der Regel handelt es sich somit um typische Einweglanzettenvorrichtungen.

Aus der DE-PS 31 11 737 ist eine Lanzettenvorrichtung der eingangs genannten Art bekannt. Diese Lanzettenvorrichtung besteht aus einem Buchsenelement und einem Steckelement, wobei das Steckelement an seinem von der Handhabe weggerichteten freien Ende eine mit einem zugespitzten Ende versehene Lanzette aufweist, die zum gezielten Eintritt in die Haut dient. Das Steckelement ist dabei mit einem sich radial erhebenden Umfangswulstwulst versehen, das am schaftartigen Steckelement in einem vorbestimmten Abstand zu einer Druckplatte angeordnet ist. Das von der Umfangswulst wegweisende Ende des Steckelements, das ebenfalls schaftartig ausgebildet ist, ist in einem entsprechend dem Schaftquerschnitt des Steckelements ausgebildeten Buchseninnenraum angeordnet, wobei aus dem freien Ende des Schaftteils des Steckelements, wie schon erwähnt, das zugespitzte Ende der Lanzette herausragt. Zur Ausführung der bestimmungsgemäßen Punktierfunktion wird bei der bekannten Lanzettenvorrichtung das Steckelement von einer Handhabe her in der Regel durch den Daumen einer die Lanzettenvorrichtung benutzenden Person in axialer Richtung verschoben, wobei die Umfangswulst über eine entsprechend in die Bahn des Steckelements im Innenraum des Buchsenelements radial hineinstehende Wulst gedrückt werden muß, wobei sich beide Wulste zur Überwindung des durch sie gebildeten Widerstandes elastisch verformen. Ist der Widerstand überwunden, schnappt die steckelementseitige Wulst über die buchsenelementseitige Wulst herüber und die Lanzettenspitze kann bestimmungsgemäß in eine vorbestimmte Tiefe durch die Haut in das Gewebe des Menschen eintreten.

Die bekannten Lanzettenvorrichtung hat wesentliche Nachteile. Diese bestehen zum einen darin, daß schon beim Einführen des Steckelements in das Buchsenelement die Lanzettenspitze ihre Sterilität verlieren kann, wenn beispielsweise die das Einführen ausführende Person versehentlich mit der Lanzettenspitze die die Eintrittsöffnung des Buchsenelements umgebende Handhabe mit der Lanzettenspitze berührt oder aber die Haut der diesen Vorgang ausführende Person versehentlich berührt wird. Zusammenfassend kann gesagt werden, daß es bei dem Einführen des Steckelements in das Buchsenelements eine Vielzahl von Möglichkeiten der Desterilisierung gibt, was gerade im Zusammenhang mit der Behandlung von HIV-Erkrankten zunehmend höchster Aufmerksamkeit zukommt.

Zwar ist bei der bekannten Vorrichtung die Lanzettenspitze in den Werkstoff der Buchse eingebettet, d.h. das Steckelement und Buchsenelement ist in einem Arbeitsgang in Form eines einzigen Spritzgußteils hergestellt, so daß die Sterilität der Lanzettenspitze soweit vollkommen gewahrt ist, in jedem Fall muß aber das Steckelement vom Buchsenelement für den bestimmungsgemäßen Einsatz der Lanzettenspitze entfernt werden und auf oben beschriebene Weise in das Buchsenelement eingeführt werden, wobei durch den Trennvorgang die Lanzettenspitze bestimmungsgemäß freigegeben und somit dem Zutritt von mikrobiologischen Keimen und Verschmutzungsbestandteilen freigegeben wird.

Ein weiterer wesentlicher Nachteil der Lanzettenvorrichtung besteht darin, daß der Auslösemechanismus, je nach Fertigkeit und Sensibilität der die Lanzettenvorrichtung betätigenden Person, mit unterschiedlicher Geschwindigkeit und somit auch mit unterschiedlichem Erfolg ausgelöst werden kann, so daß die Punktierung nicht in jedem Fall auf gewünschte Weise, d.h. mit einer entsprechenden Blutförderung aus der Austrittsöffnung, die in der Haut des Menschen geschaffen wurde, endet.

Schließlich ist es bei der bekannten Lanzettenvorrichtung von Nachteil, daß diese aufgrund des absoluten Sterilitätserfordernisses wenigstens der Lanzettenspitze nur sehr höchst aufwendig und damit kostenträchtig herstellbar ist.

Es ist Aufgabe der vorliegenden Erfindung eine Lanzettenvorrichtung der eingangs genannten Art zu schaften, die sehr einfach im Aufbau ist und kostengünstiger als bisherige Lanzettenvorrichtungen herstellbar und bereitstellbar ist, die darüber hinaus bis unmittelbar vor dem Einsatz eine absolute Sterilität der in die Haut des Menschen eindringende Teile der Lanzettenvorrichtung sicherstellt, die fortwährend eine von den individuellen Eigenschaften der Bedienungsperson unabhängige gleichmäßige Eintrittfunktion der Lanzettenspitze in die Haut des Menschen gewährleistet und die darüber hinaus nach ausgeübter Funktion sicherstellt, daß die Lanzettenspitze abgeschirmt wird, so daß auch unbeabsichtigte Verletzungen und damit einhergehende Infektionen durch an der Lanzettenspitze anhaftendes Blut vermieden werden.

Gelöst wird die Aufgabe gemäß der Erfindung dadurch, daß die Handhabe des Steckelements wenigstens teilweise als federelastischer Körper nach Art einer Membran ausgebildet ist, die die Lanzette über das Steckelement nach Überwindung eines membranseitig eingestellten Druckpunktes durch eine in axialer Richtung auf das freie Buchsenende hin einwirkende Kraft mit einem vorbestimmbaren Impuls aus der durch das Buchsenende gebildeten Ebene in die Haut eintreten läßt.

Der Vorteil der erfindungsgemäßen Lanzettenvorrichtung besteht im wesentlichen darin, daß das Steckelement fortwährend, d.h. auch im Ruhezustand, in dem die Lanzettenvorrichtung noch nicht in Funktion war, vom Buchsenelement umhüllt im Buchseninneren verbleibt, so daß auch eine irrtümliche Berührung der Lanzettenspitze und damit eine Kontamination mit mikrobiologischen Keimen oder auch sonstigen Verunreinigungen unmöglich ist. Darüber hinaus ist es von wesentlichem Vorteil, daß die Auslösung des Punktierungsvorganges völlig frei von den individuellen Eigenheiten der die Lanzettenvorrichtung benutzenden, d.h. Kriterium ist, die diese Funktion beeinflussen können, da die Bedienungsperson lediglich für die Überwindung des Druckpunktes der federelastischen Membran zu sorgen hat, d.h. nach Überwindung des Druckpunktes wird durch die Federkonstante der Membran der Impuls bestimmt, mit dem die vom Steckelement getragene Lanzettenspitze vorstößt und in die Haut des Menschen in vorbestimmte Tiefe eindringt. Schließlich ist es von Vorteil, daß die erfindungsgemääße Lanzettenvorrichtung auf einfache Weise in zwei gesondert zu fertigenden Teilen hergestellt werden kann, die nachfolgend lediglich ineinander gesteckt zu werden brauchen. Dadurch werden sehr kostenaufwendige Herstellungswerkzeuge und aufwendige Herstellungsverfahrensschritte vermieden.

Bei einer vorteilhaften Ausgestaltung der Lanzettvorrichtung ist die Membran als im wesentlichen tellerförmiger Körper ausgebildet, der im Querschnitt konkav gewölbt ist, wobei die konkave Wölbung im ungebrauchten Zustand der Lanzettvorrichtung axial vom Buchsenelement wegstehend ausgebildet ist. Zwar ist es grundsätzlich möglich, die Membran auf beliebige geeignete Weise auszubilden und herzustellen, vorteilhaft, weil die Herstellungskosten der Lanzettenvorrichtung verbilligend ist es jedoch, die Membran und das schaftartig ausgebildete Steckelement als einstückiges Formteil auszubilden, so daß prinzipiell die Lanzettenvorrichtung nur aus dem Steckelement mit Membran und dem Buchsenelement, in der das Steckelement aufgenommen wird, besteht.

Bei einer anderen vorteilhaften Ausgestaltung der Lanzettenvorrichtung ist das Steckelement an seinem Schaftteil mit Vorsprüngen versehen, die mit entsprechenden Vorsprüngen, die im Innenraum des Brückenelementes angeordnet sind, nach Einführen des Steckelementes in das Buchsenelement gegenseitig verrastend zusammenwirken, so daß das Steckelement nicht mehr aus dem Buchsenelement, ohne die Teile zu zerstören, entfernt werden kann, was wiederum den Vorteil hat, daß beide Elemente eine funktionsgerechte Einheit bilden, die einheitlich gelagert und zur Ausführung der bestimmungsgemäßen Punktierungsfunktion gehandhabt werden kann.

Bei einer noch anderen vorteilhaften Ausgestaltung der Lanzettenvorrichtung weist das Steckelement an seinem freien Ende ein die daraus hervorstehende Lanzettenspitze steril umhüllendes Abdeckelement aufweist. Zwar ist es grundsätzlich möglich, daß die Lanzettenvorrichtung ohne dieses Abdeckelement in seinen Einzelheiten nach der Herstellung auf geeignete Weise sterilisiert und in eine luftdichte Umhüllung eingefügt werden kann, so daß bei Gebrauch lediglich die Umhüllung aufgetrennt und die Lanzettenvorrichtung herausgenommen zu werden braucht, es hat sich jedoch als vorteilhaft erwiesen, aus Kostengründen und auch aus Gründen der einfacheren Handhabbarkeit lediglich ein Abdeckelement im vorbeschriebenen Sinne vorzusehen, das die Lanzettenspitze steril abdeckt und das Abdeckelement unmittelbar vor Gebrauch der Lanzettenvorrichtung von der Lanzettenspitze zu entfernen.

Bei einer noch anderen weiteren vorteilhaften Ausgestaltung der Lanzettenvorrichtung sind das Steckelement und das Abdeckelement als einstükkiges Formteil ausgebildet, mit der Folge, daß diese Ausgestaltung in einem Arbeitsgang und somit kostengünstig herstellbar ist.

Um das Abdeckelement für den Punktierungsvorgang mit der Lanzettenvorrichtung leicht und schnell vom Steckelement entfernen und so die Lanzettenspitze freizulegen, ist das Steckelement vorzugsweise im Bereich des Anschlusses an das Abdeckelement mit einer Sollbruchstelle versehen. Die Sollbruchstelle selbst gestatten ein schnelles Entfernen des Abdeckelements, vorzugsweise dann, wenn die Sollbruchstelle vorteilhafterweise durch eine radiale Verjüngung des Anschlußbereiches gebildet wird, so daß das Abdeckelement gegenüber dem Steckelement lediglich zu seiner Entfernung geringfügig um die Achse gedreht zu werden braucht und so die Verbindung zwischen Steckelement und Abdeckelement, d.h in der Verjüngung des Anschlußbereiches bricht.

Vorzugsweise ist das aus dem Buchsenelement bei mit diesem verrasteten Steckelement heraustretende Teil des Abdeckelementes als Griffelement ausgebildet, wobei durch diese Art des Herausstehens des Abdeckelements bzw. des dort gebildeten Griffelements sichergestellt wird, daß die Entfernung des Abdeckelementes vom Steckelement unabhängig von der Betätigung des fehlerelastischen Membranelements ist, d.h. die Entfernung des Abdeckelements über das Griffelement generell ohne vorherige Betätigung der Lanzettenvorrichtung möglich ist.

Die Handhabe des Buchsenelements selbst ist vorzugsweise als im wesentlichen tellerförmiger Körper ausgebildet, auf dem bei mit dem Buchsenelement verrasteten Zustand des Steckelementes die Handhabe des Steckelementes aufliegt, wobei beide Handhaben im miteinander zusammengefügten Zustand quasi eine Einheit bilden, die auf einfache Weise, anders als die bekannte Lanzettenvorrichtung, einfach und sicher handhabbar ist.

Grundsätzlich kann der Buchsenkörper des Buchsenelementes der Lanzettenvorrichtung auf beliebige geeignete Weise ausgebildet sein, beispielsweise mit einem kreisförmigen Querschnitt. Es hat sich jedoch als vorteilhaft herausgestellt, daß der Buchsenkörper des Buchsenelementes einen im wesentlichen ovalen Querschnitt aufweist, was gleichermaßen für den im Buchsenkörper gebildeten Innenraum gilt. Ein so ausgebildeter Buchsenkörper gestattet ohne weiteres den Durchtritt des Steckelements mit integral an diesem ausgebildeten Abdeckelement mit Griffelement, das dann einen vorzugsweise ebenfalls im wesentlichen ovalen Querschnitt aufweisen kann, d.h. das Griffelement weist faktisch zwei flügelartige Teile auf, die das Abdrehen des Abdeckelements zur Freisetzung der Lanzettenspitze auf einfache Weise gestatten.

Grundsätzlich kann gesagt werden, daß die Lanzettenvorrichtung aufgrund ihres Gesamtaufbaus durchaus mehrfach verwendbar ist, da durch geeignete Gegenlage der Lanzettenspitze auf einem widerstandsfähigen Untergrund die Membran wiederum nach Überwindung ihres voreingestellten Druckpunktes in ihre Ausgangsposition vor dem erfolgten Punktieren zurückschnappen kann. Dieses kann beispielsweise dann erforderlich sein, wenn bei ein und demselben Probanden aufgrund bestimmter Hautstrukturen Blutgefäße nicht in ausreichendem Maße getroffen worden sind und somit nicht ausreichend Blut aus der Hautöffnung austritt.

Es können aber auch bei einer bestimmten Ausführungsform der Lanzettenvorrichtung, die bewußt nur einen einmaligen Gebrauch ermöglichen soll, vorteilhafterweise mit dem Steckelement und dem Buchsenelement zusammenwirkende Blockierungsmittel vorgesehen werden, die durch gegenseitig wirkenden sperrenden Eingriff nach erfolgter Auslösung eine weitere Auslösung der Lanzettenvorrichtung verhindern.

Die Erfindung wird nun unter Bezugnahme auf die nachfolgenden schematischen Zeichnungen anhand eines Ausführungsbeispieles eingehend beschrieben. Darin zeigen:
Fig. 1 in der Seitenansicht eine aus Steckelement und Buchsenelement im zusammengefügten Zustand bestehende Lanzettenvorrichtung in vergrößerter Darstellung,
Fig. 2 einen Schnitt durch die in Fig. 1 dargestellte Lanzettenvorrichtung entlang der Linie A - B,
Fig. 3 im Schnitt ein Steckelement mit an seinem freien Ende angeordneten Abdeckelement mit damit verbundenem Griffelement,
Fig. 4 eine Darstellung von Fig. 3 in der Seitenansicht, jedoch in gegenüber der Fig. 3 um die Steckelementachse um 90° gedrehtem Zustand,
Fig. 5 eine Lanzettenvorrichtung gemäß der Darstellung von Fig. 1 im Schnitt, jedoch um die Steckelementachse gegenüber der Darstellung von Fig. 1 um 90° gedreht, und
Fig. 6 eine Darstellung gemäß Fig. 5 im Schnitt, jedoch mit von der Lanzettenspitze entferntem Abdeckelement und in die Haut eines Menschen eingedrungenem Zustand der Lanzettenspitze beim bestimmungsgemäßen Punktierungsvorgang.

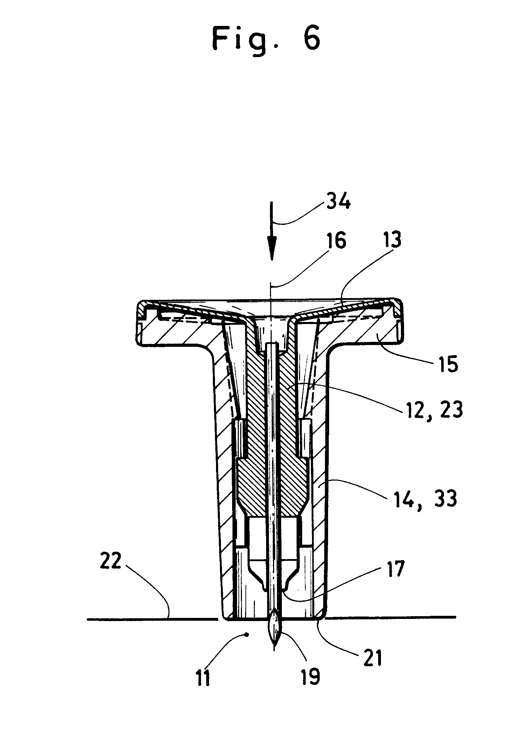

Die Lanzettenvorrichtung 10 besteht im wesentlichen aus einem Steckelement 12 sowie einem Buchsenelement 14, die im ineinander zusammengesteckten Zustand zusammen mit einer Lanzette 18 die gesamte Vorrichtung bilden. Das Steckelement 12 weist ein im wesentlichen zylinderförmiges Schaftteil 23 auf, in dem im wesentlichen axial zur Steckelementachse 16 eine Lanzette 18, die in der Regel aus einem gewebeverträglichen Edelstahl besteht, eingebettet ist. Darüber hinaus weist das Steckelement 12 an seinem Schaftteil Vorsprünge 23, 24 auf, die radial vorstehend ausgebildet sind. Die Vorsprünge 24, 25 wirken mit Vorsprüngen 26, 27 zusammen, die im Innenraum 28 des Buchsenelementes 14 angeordnet sind, was im einzelnen noch weiter unten beschrieben wird. Am freien Ende 17 des Steckelements 12, vergleiche insbesondere Fig. 6, steht die Lanzettenspitze 19 aus dem Steckelement 12 während der bestimmungsgemäß vorzunehmenden Punktierungsfunktion heraus. Vor Aufnahme der bestimmungsgemäßen Punktierungsfunktion wird jedoch ein unmittelbar an das freie Ende 17 des Steckelements 12 anschließendes Abdeckelement 29 vorgesehen, das bei der in den Figuren dargestellten Ausgestaltung der Lanzettenvorrichtung 10 als einstückiges Formteil mit dem Steckelement 12 ausgebildet ist. Somit wird die Lanzettenspitze 19 steril vom Abdeckelement 29 umhüllt.

Die Lanzette 19 weist, anders als die im Stand der Technik bekannten Lanzetten, keine Verdrehsicherung auf, beispielsweise durch Abflachung bestimmter Teile der Lanzette 18. Vielmehr wird bei der Vorrichtung aufgrund des zur Herstellung des Steckelementes 12 verwendeten Werkstoffs infolge der beim Schrumpfungsvorgang des Werkstoffs nach der Herstellung aufgebauten Reibungskraft diese derart bemessen, daß bei abgeschlossener Schrumpfung keine Drehung der Lanzette 18 mehr erfolgt, was zu einer beträchtlich vereinfachten und gegenüber den bekannten Lanzettenvorrichtungen kostengünstigeren Lösung führt.

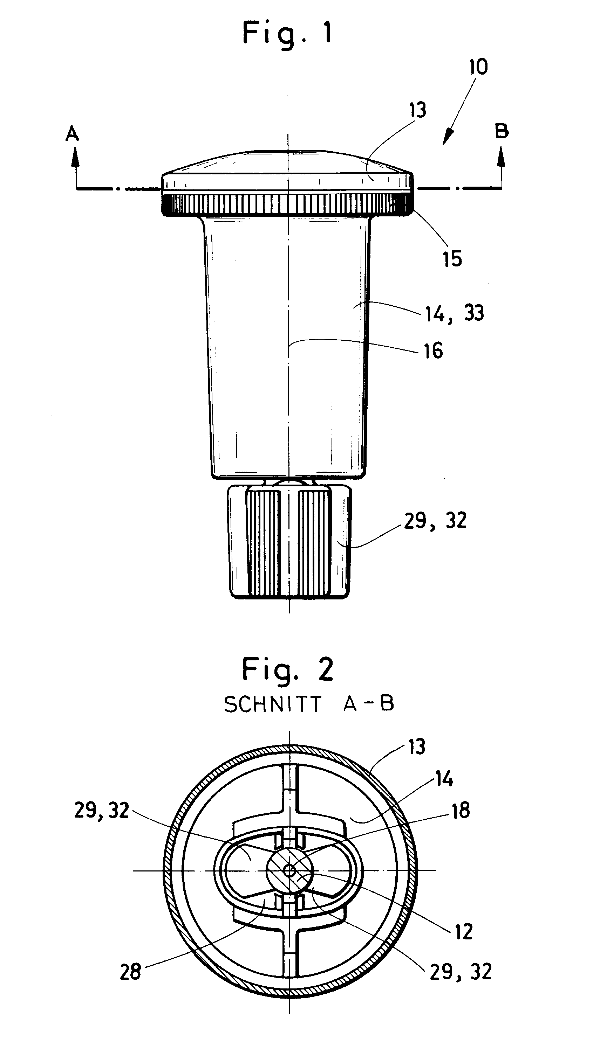

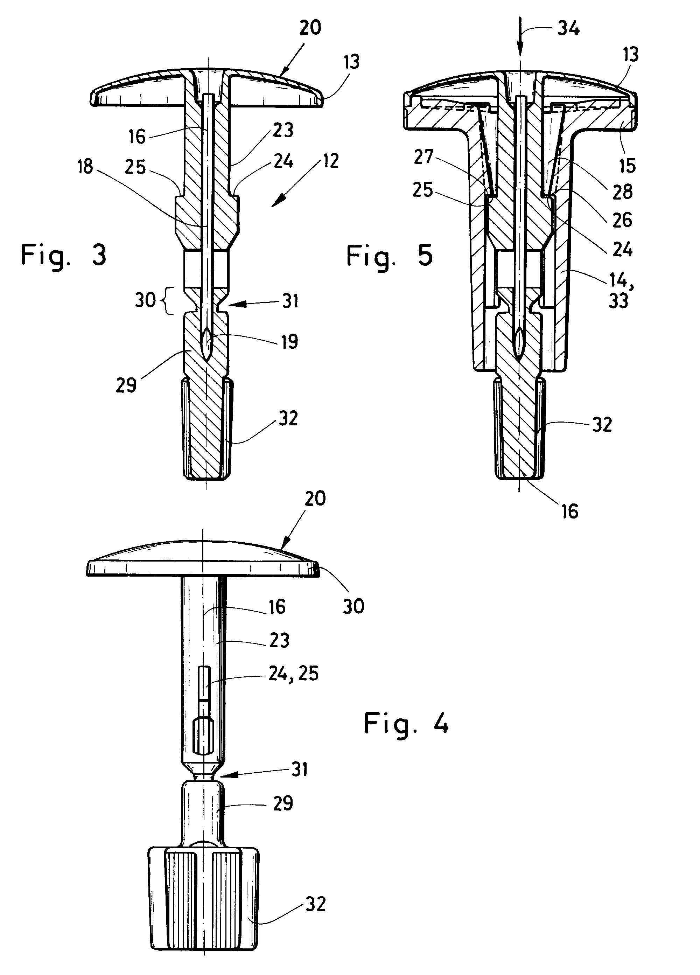

Der Anschlußbereich 30 zwischen Steckelement 12 und Abdeckelement 29 ist, vergleiche insbesondere die Figuren 3 und 4, mit einer Sollbruchstelle 31 versehen, die durch eine radiale Verjüngung des Anschlußbereiches 30 gebildet wird. Der verlängerte Teil des Abdeckelementes 29, der im verrasteten Zustand des Buchsenelementes 14 und des Steckelementes 12 aus dem Buchsenkörper 33, vergleich Fig. 1, heraussteht, bildet ein im Querschnitt ovales bzw. doppelflüglig ausgebildetes Griffelement 32. Durch Drehen des Griffelementes 32 gegenüber dem Schaftteil 23 des Steckelementes 12 kann die Sollbruchstelle 31, die durch die radiale Verjüngung des Anschlußbereiches 30 gebildet wird, gebrochen werden und das Abdeckelement 29 von der Lanzettenspitze 19 entfernt werden.

Die Handhabe 13 des Steckelements 12, die am der Lanzettenspitze 19 entgegengesetzten Ende des Schaftteils 23 ausgebildet ist, wird durch einen federelastischen Körper nach Art einer Membran 20 ausgebildet. Die Membran 20 wird durch einen im wesentlichen tellerförmigen Körper gebildet, der im Querschnitt konkav gewölbt ist und mit seinem höchsten Wölbungspunkt von Schaftteil 23 in Ruhestellung wegweist, vergleiche die Figuren 1 bis 5.

Das Buchsenelement 14 weist ebenfalls eine Handhabe 15 auf, die ebenfalls als im wesentlichen tellerförmiger Körper ausgebildet ist. Die Handhabe 15 weist an ihrem Umfang einen umlaufenden Rezeß auf, der derart ausgebildet ist, daß die Handhabe 13 des Steckelements 12 mit ihrem umlaufenden Seitenrand in diesem Rezeß aufliegt, vergleiche die Figuren 1 und 5. Der Buchsenkörper 33 des Buchsenelementes 14 weist sowohl äußerlich als auch innerlich einen im wesentlichen ovalen Querschnitt auf, d.h. der Innenraum 28 des Buchsenelementes 14, vergleiche Fig. 2, ist ebenfalls oval im Querschnitt ausgebildet. Wie oben schon erwähnt, sind im Innenraum 28 des Buchsenelementes 14 Vorsprünge 26, 27 ausgebildet, die nach Einführung des Steckelementes 12, vergleiche Fig. 5, in das Buchsenelement 14 gegenseitig verrastend mit den Vorsprüngen 24, 25 des Schaftteils 23 des Steckelementes 12 zusammenwirken.

Für den bestimmungsgemäßen Gebrauch der Lanzettenvorrichtung 10 zur Ausführung einer Punktierung der menschlichen Haut 11 wird, was noch in den Bereich der Fertigung gehört, zunächst das Steckelement 12 in den Innenraum 28 des Buchsenelementes 14 eingeführt, so daß die steckelementseitigen Vorsprünge 24, 25 mit den buchsenselementseitigen Vorsprüngen 26, 27 verrasten. Bedingt durch die Federvorspannung der federelastischen Membran 13, die im wesentlichen durch die Handhabe 13 gebildet wird, kann das Steckelement 12 sich in Richtung der Achse 16 normalerweise nicht bewegen. Nach Entfernen des Abdeckelementes 29 von der Lanzettenspitze 19, wie oben beschrieben, steht die Lanzettenspitze 19 frei im Innenraum 28 des Buchsenselementes 14, ohne die durch das freie Ende 21 des Buchsenelementes 14 gebildeten Ebene 22 zu durchstoßen. Nach Ausübung einer Kraft in Richtung des Pfeiles 34, die zur Überwindung eines membranseitig eingestellten Druckpunktes in axialer Richtung in Richtung des Pfeiles auf das freie Buchsenende 21 hin, die durch eine Person ausgeübt wird, wird durch den federelastischen Körper ein vorbestimmbarer Impuls auf das Steckelement 12 und somit auf die darin eingebettete Lanzette 18 ausgeübt, die dann mit diesem Impuls durch die Ebene 22 hindurchtritt und in die Haut 21 eines Menschen, vergleiche Fig. 6, in vorbestimmbarer Tiefe eintritt. Die vorbestimmbare Tiefe des Eintritts der Lanzettenspitze 19 in die Haut 21 wird durch die konstruktive Ausgestaltung bzw. Festlegungen der axialen Verschiebbarkeit der Membran 20 festgelegt.

Der erfindungsgemääß verwendete Werkstoff ist vorzugsweise Polyäthylen, Polypropylen oder beliebiger andere geeignete spritzfähige Kunststoffwerkstoffe, die zudem noch gewebeverträglich und gesundheitsunbedenklich sind.

### Bezugszeichenliste

10 Lanzettenvorrichtung
11 Haut
12 Steckelement
13 Handhabe
14 Buchsenelement
15 Handhabe
16 Buchsenachse / Steckelementachse
17 freies Ende
18 Lanzette
19 Lanzettenspitze
20 Membran
21 freies Ende
22 Ebene
23 Schaftteil
24 Vorsprung
25 Vorsprung
26 Vorsprung
27 Vorsprung
28 Innenraum
29 Abdeckelement
30 Anschlußbereich
31 Sollbruchstelle
32 Griffelement
33 Buchsenkörper
34 Pfeil

## Patentansprüche

1. Lanzettenvorrichtung zum Punktieren der Haut von Säugetieren, insbesondere des Menschen, umfassend ein mit einer Handhabe versehenes Steckelement sowie ein mit einer Handhabe versehenes Buchsenelement zur inneren axial verschiebbaren Aufnahme des Steckelements, wobei das Steckelement an seinem von der Handhabe weggerichteten freien Ende eine mit einem zugespitzten Ende versehene Lanzette zum gezielten Eintritt in die Haut aufweist, dadurch gekennzeichnet, daß die Handhabe (13) des Steckelements (12) wenigstens teilweise als federelastischer Körper nach Art einer Membran (20) ausgebildet ist, die die Lanzette (18) über das Steckelement (12) nach Überwindung eines membranseitig eingestellten Druckpunktes durch eine in axialer Richtung auf das freie Buchsenende (21) hin wirkende Kraft mit einem vorbestimmbaren Impuls aus der durch das Buchsenende (21) gebildeten Ebene (22) in die Haut (11) eintreten läßt.

2. Lanzettenvorrichtung nach Anspruch 1, dadurch gekennzeichnet, daß die Membran (20) als im wesentlichen tellerförmiger Körper ausgebildet ist, der im Querschnitt konkav gewölbt ist.

3. Lanzettenvorrichtung nach einem oder beiden der Ansprüche 1 oder 2, dadurch gekennzeichnet, daß die Membran (20) und das schaftartig ausgebildete Steckelement (12) als einstückiges Formteil ausgebildet sind.

4. Lanzettenvorrichtung nach einem oder mehreren der Ansprüche 1 bis 3, dadurch gekennzeichnet, daß das Steckelement (12) an seinem Schaftteil (23) Vorsprünge (24, 25) aufweist, die mit entsprechenden Vorsprüngen (26, 27) die im Innenraum (28) des Buchsenelementes (14) angeordnet sind, nach Einführung des Steckelementes (12) in das Buchsenelement (14) gegenseitig verrastend zusammenwirken.

5. Lanzettenvorrichtung nach einem oder mehreren der Ansprüche 1 bis 4, dadurch gekennzeichnet, daß das Steckelement (12) an seinem freien Ende (17) ein die daraus hervorstehende Lanzettenspitze (19) steril umhüllendes Abdeckelement (29) aufweist.

6. Lanzettenvorrichtung nach Anspruch 5, dadurch gekennzeichnet, daß das Steckelement (12) und das Abdeckelement (29) als einstückiges Formteil ausgebildet sind.

7. Lanzettenvorrichtung nach einem oder beiden der Ansprüche 5 oder 6, dadurch gekennzeichnet, daß das Steckelement (12) im Bereich (30) des Anschlusses an das Abdeckelement (29) mit einer Sollbruchstelle (31) versehen ist.

8. Lanzettenvorrichtung nach Anspruch 7, dadurch gekennzeichnet, daß die Sollbruchstelle (31) durch eine radiale Verjüngung des Anschlußbereiches (30) gebildet wird.

9. Lanzettenvorrichtung nach einem oder mehreren der Ansprüche 5 bis 8, dadurch gekennzeichnet, daß das aus dem Buchsenelement (14) bei mit diesem verrasteten Steckelement (12) herausstehende Teil des Abdeckelementes (29) als Griffelement (32) ausgebildet ist.

10. Lanzettenvorrichtung nach einem oder mehreren der Ansprüche 1 bis 9, dadurch gekennzeichnet, daß die Handhabe (13) des Buchsenelementes (14) als im wesentlichen tellerförmiger Körper ausgebildet ist, auf dem bei mit dem Buchsenelement (14) verrasteten Zustand des Steckelementes (12) die Handhabe (13) des Steckelementes (12) aufliegt.

11. Lanzettenvorrichtung nach einem oder mehreren der Ansprüche 1 bis 10, dadurch gekennzeichnet, daß der Buchsenkörper (33) des Buchsenelementes (14) einen im wesentlichen ovalen Querschnitt aufweist.

12. Lanzettenvorrichtung nach einem oder mehreren der Ansprüche 9 bis 11, dadurch gekennzeichnet, daß das Griffelement (32) einen im wesentlichen ovalen Querschnitt aufweist.

13. Lanzettenvorrichtung nach einem oder mehreren der Ansprüche 1 bis 12, dadurch gekennzeichnet, daß das Steckelement (12) und das Buchsenelement (14) miteinander zusammenwirkende Blockierungsmittel aufweisen, die durch gegenseitig wirkenden sperrenden Eingriff nach erfolgter Auslösung eine weitere Auslösung verhindern.
